Europäisches Patentamt

European Patent Office

Office européen des brevets

(19)

(11)  EP 0 714 506 B1

(12)  EUROPEAN PATENT SPECIFICATION

(45) Date of publication and mention
of the grant of the patent:
**10.11.2004  Bulletin 2004/46**

(51) Int Cl.[7]: **G01N 1/28**, G01N 25/48,
G01N 33/48, G01N 35/04,
G01N 33/49

(21) Application number: **94925895.8**

(22) Date of filing: **16.08.1994**

(86) International application number:
**PCT/US1994/009226**

(87) International publication number:
**WO 1995/005590 (23.02.1995 Gazette 1995/09)**

(54) **METHOD AND INSTRUMENT FOR AUTOMATICALLY PERFORMING ANALYSIS RELATING TO THROMBOSIS AND HEMOSTASIS**

METHODE UND GERÄT ZUR AUTOMATISCHEN DURCHFÜHRUNG VON ANALYSEN BEZÜGLICH THROMBOSE UND BLUTGERINNUNG

PROCEDE ET INSTRUMENT DESTINES A EFFECTUER AUTOMATIQUEMENT UNE ANALYSE DES PROPRIETES DE THROMBOSE ET D'HEMOSTASE

(84) Designated Contracting States:
**AT BE CH DE DK ES FR GB GR IE IT LI LU MC NL
PT SE**

(30) Priority:  **16.08.1993  US 107381**

(43) Date of publication of application:
**05.06.1996  Bulletin 1996/23**

(73) Proprietor: **bioMérieux, Inc.
Treyburn Durham, NC 27712 (US)**

(72) Inventors:
• **FISCHER, Timothy, J.
Raleigh, NC 27613 (US)**
• **CALLAHAN, Janet, B.
Chapel Hill, NC 27516 (US)**
• **BRAUN, Paul, J.
Durham, NC 27712 (US)**
• **GIVENS, Thomas, B.
Rougemont, 27572 (US)**
• **HULETTE, William, C.
Hillsborough, NC 27278 (US)**
• **HOFFMAN, Julie, F.
Ypsi, MI 48197 (US)**
• **LINK, John, G.
Durham, NC 27712 (US)**
• **SWOPE, Charles, H.
Raleigh, NC 27613 (US)**

(74) Representative: **Prins, Hendrik Willem et al
Arnold & Siedsma,
Advocaten en Octrooigemachtigden,
Sweelinckplein 1
2517 GK Den Haag (NL)**

(56) References cited:
**US-A- 4 483 927          US-A- 4 517 160
US-A- 4 695 430          US-A- 4 958 295
US-A- 4 971 913          US-A- 5 100 622**

# EP 0 714 506 B1

## Description

This application is related to the following copending U.S. Patent Applications and issued patents, which are owned by the Assignee of the present Application, and the disclosures of all but No. 6 are incorporated herein by reference:

(1) Serial No. 07/443,952, to Swope et al., titled "Multichannel Optical Monitoring Systems", now U.S. Patent 5,002,392;
(2) Serial No. 07/443,956, to Karp et al., titled "Cuvette and Linear Drive Mechanism Therefor", now U.S. Patent 5,040,894; and
(3) Serial No. 07/443,954, to Hoffman et al., titled "Apparatus and Method for Cleaning Reagent Delivery Probes", now U.S. Patent 4,989,623.
(4) Serial No. 07/443,784, to Karp et al., titled, "Cuvette", now U.S. Patent Des. 325,090.
(5) Serial No. 07/674,957, to Keiter et al., titled, "Heated Liquid Sampling Probe for an Automated Sampling Apparatus," now U.S. Patent 5,178,019.
(6) Serial No. 07/916,712, to Lewis et al., titled , "Cassette and Cuvette Loading Mechanism".
(7) Serial No. 07/896,579, to Haugen, titled, "Method for Scanning Photodiodes".
(8) Serial No. 07/443,953, to Driscoll, titled, "Method of Monitoring Reagent Delivery in a Scanning Spectrophotometer," now U.S. Patent 5,068,181.
(9) Serial No. 07/833,950, to Fischer et al., titled, "Method and Instrument for Automatically Performing Analysis Relating to Thrombosis and Hemostasis."
(10) Serial No. 07/443, 951, to Fischer et al., titled, "Method and Instrument for Automatically Performing Analysis Relating to Thrombosis and Hemostasis."

This invention relates to a novel, fully automated spectrophotometric analyzer and the method used for assaying for thrombosis and hemostasis properties of blood samples. The analyzer tests samples in a fully randomized format, and is fully automated in the areas of specimen handling, sample preparation, optical inspection, signal processing and quality assurance/control for imprecision and bias allowing for numerous assays to be performed on a single sample. The assays vary considerably in nature and fall into three categories: clot based, chromogenic, and immunological. No single automated methodology or instrumentation currently exists that successfully performs all of these types of assays concurrently.

## BACKGROUND OF THE INVENTION

Thrombosis and hemostasis testing is the in vitro study of the ability of the blood to form clots and to break clots in vivo. As thrombotic and hemostasis pathways form a part of very important disease states ranging from hemophilia to strokes and heart attacks, the testing of a patients capabilities in thrombosis and hemostasis is a critical diagnostic tool. Should a patients ability to form clots in vitro fall outside of the established norm, or should certain markers be out of the normal range, the serum or plasma sample is further assayed to determine the reason for the problem. These assays are in standard use in all hospital laboratories.

Coagulation assays began, and are still done in many instances, in a test tube using hand methods. Early on, the goal was to determine if a patients blood sample would clot after certain materials were added. It was later determined that the amount of time it took for the sample to clot was related to congenital or acquired disorders. This type of testing is extremely dependent on the laboratory technologist, and so, some form of standardization was seen to be needed. As technology improved and stronger correlations between in vivo conditions and in vitro assays were established, semi-automatic coagulation analyzers began to appear.

These coagulation analyzers primary usefulness is to remove the subjectiveness in determining the exact second a clot forms in a sample in vitro. However, these analyzers did and do not have the automation required to remove variability associated with sample preparation. Furthermore, advances in clinical thrombosis and hemostasis assays resulted in the development of new types of assays that aided in the diagnosis and treatment of a patient and semi-automated coagulation analyzers seldom possess the ability to perform more than one assay at a time. This is because reagent pathways are dedicated to a single reagent, resulting in a limited number of assays that can be performed on each instrument. Generally, the semi-automated instrumentation performs one test in a batch mode, maintaining one profile of temperature vs. time for each different type of assay.

Semi-automated analyzers also require the technician to manually deliver the plasma sample. A new sampling device, generally a pipette tip, is used for each specimen to eliminate plasma cross-contamination between samples. Using a common sampling means for reagents and samples requires novel approaches to eliminate cross contamination of samples and reagents.

In order to have the next generation of analyzers, fully automated analyzers must be developed to be able to use the same sampling device for all specimens and to have common pathways for delivery of multiple reagents, and to provide a universal time and temperature profile compatible with a multitude of assays.

Additionally, any complex computations needed to be performed for an assay are done by an operator when semi-automated analyzers are used. Differences in operators techniques in analyzing data lead to increased levels of inaccuracy of the data. Another feature needed to improve coagulation testing is improved and standardized data analysis techniques to obtain the desired performance characteristics from inter and intra laboratory comparisons, which would result in a higher standard of care for the patient.

Quality control and system monitoring of the semi-automated coagulation analyzers are primitive and inadequate when compared to the state of the art.

The next generation of analyzers, a fully automated thrombosis and hemostasis analyzer, requires a statistically controlled, on-line quality assurance program that monitors the system integrity, as the analysis are being performed. This program must not only identify failures after they have occurred, but predict potential failures before they occur.

Another area of the clinical laboratory, the clinical chemistry laboratory, has had fully automated analyzers for a number of years. The tests performed and the types of reactions read, including colorimetric, fluorescent and luminescent measurement, are substantially different and have endpoints that are easier to detect than do coagulation-based tests, those performed in the coagulation laboratory. The same progress towards full automation has not been seen in the coagulation laboratory as in the clinical chemistry laboratory.

In general, the basic tests or assays performed in the coagulation laboratory using plasma, serum or whole blood include performing the Partial Thromboplastin Time ("PTT"), the Prothrombin Time ("PT"), the Activated Partial Thromboplastin Time ("APTT"), testing for deficiencies in Factors such as Factors II, V, VII, VIII, IX, XI, XII and others, and chromogenic and immunological testing for thrombosis or hemostasis markers. These, among others, have proven to be much more difficult to do on automated equipment than have the clinical chemistry tests. This is because the tests run in the coagulation laboratory usually: (1) involve unique time/temperature profiles; (2) are extremely sensitive to both reagent and plasma carryover; (3) require unique data analysis; and (4) have unique quality control requirements.

A method for automatically performing a variety of coagulation-related assays and a fully automated coagulation analyzer is needed to perform a host of assays in a totally random format that would expedite patient diagnosis. It must have the ability to control the sample preparation stages of an in vitro assay; to perform all thrombosis and hemostasis assays using a common temperature profile; to measure the reaction that occurs when the appropriate materials are added; and to determine both the immediate response as well as to provide mathematical tools for calculating complex results. This entire process should be monitored using an on-line quality control package that is designed to minimize imprecision associated with random error and to minimize bias associated with error due to the system itself, systemic error.

This type of fully automated coagulation analyzer would provide more accurate results that in turn would allow for quicker and more accurate diagnosis of current or predicted illness, thereby allowing for better treatment of the patient.

SUMMARY OF THE INVENTION

The present invention includes a method for automatically assaying for hemostasis and thrombosis parameters in a plasma, serum or whole blood sample according to claim 1.

The present invention also provides a fully automated coagulation analyzer that responds to all of the needs stated above. The linear, spectrophotometric analyzer performs multiple hemostasis and thrombosis assays on samples of serum, plasma or whole blood in a totally random order. It is fully automated in terms of specimen handling, sample preparation, optical inspection, signal processing, and total quality control for imprecision and bias. All hemostasis and thrombosis assays performed on the analyzer have been designed to share a common temperature profile, thereby allowing the random performance of assays. Each of these functions are also internally controlled through quality assurance programming. Each assay is defined by an assay definition file ("adf") allowing for flexibility in method definition. This flexibility is required in order to obtain the unique performance characteristics for each assay. Improvements in linearity, accuracy and the minimization of bias can be achieved by optimization of adf parameters.

BRIEF DESCRIPTION OF THE DRAWINGS

Figure 1 - The relative signal at various wavelengths is shown for two samples: a sample with mild Hemolysis, and a sample with Gross Hemolysis. Wavelengths of 515nm and 535nm are identified. The actual data collected is represented by data markers and these data markers are connected for each sample.

Figure 2 - The temperature of a sample is shown for a PT assay definition and an APTT assay definition as a function of time. Each sample/reagent delivery location is identified. The acceptable temperature ranges at each instant in the temperature profile are identified by "zones."

Figure 3 shows the relative absorbance rates of a dye sample, serially diluted. A Least Squares regression line is also shown, and its goodness-of-fit measure $r^2$.

Figure 4 shows the actual data points representing the clot times recovered for a serially diluted reference, and (1) the traditional curve and (2) the new curve fit through those points.

Figure 5 shows a smoothed signal, smoothed first derivative of that signal, and smoothed second derivative for data collected from a normal APTT assay. The location of the maximum of the second derivative (the clot time), is identified.

DETAILED DESCRIPTION OF THE INVENTION

The needs of the clinical coagulation laboratory for a fully automated coagulation analyzer have been met with this invention. Provided is an optical evaluation instrument and method that can handle a high throughput of patient samples with a high degree of versatility, adaptability and reliable automation. This is walk-away automation once patient samples still sealed in the original evacuated collection tube are loaded into the system.

The present invention includes a method for automatically assaying for hemostasis and thrombosis parameters as described above, in a plasma, serum or whole blood sample in a holding device, such as an evacuated collection tube, that can be stoppered. The assays may be performed in either a batch or random fashion, wherein the same assay or test is performed on all samples, multiple different assays are performed on each sample and a different assay is performed on each sample. This is accomplished by providing an integrated programming input means for identifying the sample and scheduling one or more hemostasis and thrombosis-related assays to be performed on the sample in any order. The programming means will accept and will allow the assays to be performed in a random format, i.e. in any order.

Next in the method is providing a specimen handling means for automatically transferring an aliquot of the sample from a holding device or a collection tube, with or without a stopper, to a test well in a cuvette. Because of various issues regarding the exposure of laboratory workers to blood borne contagious disease, being able to provide a specimen handling means to piece the septum or stopper without a human present is a definite asset in a clinical instrument or method.

Also, in the method is providing a sample preparation means for automatically adding the reagents needed for an assay to measure hemostasis or thrombosis parameters to the sample in the test well at a specified time and temperature, accommodating a universal thermal profile, to obtain a reaction, and wherein the order of the reagents added can be in a random format eliminating the need for batch analysis. This methodology is needed because each assay or test performed in the coagulation laboratory is done with different time and temperature needs. The present method provides for a common universal thermal profile wherein each assay or test can be done within its particular parameters. The method provides for a heating and cooling track where each sample moves forward, but each at the speed needed for the assay to be performed, and the means for automatically adding the reagent needed at the proper temperature. Once the proper reagents are added at the proper time and temperature, the reaction begins.

Next, the method provides a detecting means for detecting the reaction in the well, and takes the result of said detection to mathematically compute data. This data, or the raw result of the assay or test, is provided a processing means wherein the computed data is evaluated to determine the change in or the magnitude of the reaction in the well, and when required, to transform the data into a diagnostically useful result.

Simultaneously with all of steps above, the method provides a quality assurance means for monitoring the performance of the method and evaluating the validity of the reported data for the sample, thereby automatically performing hemostasis and thrombosis-related assays and determining and reporting results on hemostasis and thrombosis parameters in the sample.

A preferred way of performing the above described method of testing a large number of samples in a random order is done with the following instrument.

## I. Overview of the Automated Analyzer

The analyzer is fully automated in terms of specimen handling, sample preparation, optical inspection, signal processing and total quality control for imprecision and bias, and a quality assurance program.

A. The Specimen Handling Segment.

The specimen handling segment of the analyzer is divided into four basic components consisting of positive patient identification of the sample, screening for preanalytical variables, the ability to do closed container sampling and the ability to continuously supply cuvette wells for sample evaluation.

In greater detail, the specimen handling segment consists of:

1) a means for storing and continuously supplying a plurality of cuvettes to be used in the assays, each cuvette containing a plurality of reaction wells;

2) the optically readable code, such as a bar code or other coding, present on or incorporated into the sample collection tube or holding device containing a sample of serum, plasma or whole blood, is a patient identification and tracking device, and is also used in assay entry and automatic tracking of the assay during sample evaluation;

3) a means for the screening of and evaluating preanalytical variables prior to the beginning of any assay, such as hemolysis, bilirubin and lipemia; and

4) a sample insertion station including a means for automatically aspirating sample from the sample collection tube or holding device with or without a stopper and for automatically dispensing the aspirated sample into a reaction well of a cuvette.

B. The Sample Preparation Segment.

The sample preparation segment of the analyzer consists of four components: the means for defining unique reagent transferring sequences for each assay, random access ability, or the ability for a probe to aspirate reagent from a reagent container and dispense it into a predetermined cuvette well in any order and to aspirate and deliver different plasmas without cross contamination; the universal profile testing method; a means for performing auto dilutions of the sample; and a means for monitoring reagents and samples.

In greater detail, the sample preparation segment consists of:

1) an assay definition file ("adf") that allows for flexibility in how the reagents and plasmas are delivered. This flexibility is defined in terms of aspiration and dispense velocities, temperature, time out (delay) or timing sequences;

2) a reagent station, including a sample preparation means having the ability to randomly aspirate selected amounts of selected reagents from selected reagent containers as needed, and for dispensing the aspirated reagents into a reaction well of a cuvette according to the directions given in a programmed test for the sample in that reaction well. Each well of the cuvette may be programmed to have a different assay performed, and the reagent and sample in the reaction well forms a reaction volume which exhibits optical characteristics to be monitored by the analyzer;

3) a means for providing a universal temperature profile for the different assays programmed on the analyzer, which is an arrangement for temperature regulation of a fluid sample in a cuvette transported through various stations of the automated system for optically monitoring the sample in the cuvette, comprising: a means for transporting the cuvette through the various stations of the sample and reagent delivery system and optical monitoring system, the sample temperature being controlled by the system; cooling means for cooling the sample in the first portion of the profile; heating means for heating the sample in the third portion of the profile in such a way as to maintain the sample temperature within base tolerance constraints; and a second section providing a means of providing a temperature ramp that defines the sample transition from the initial cool temperature to final warm temperature;

4) a means for automatically diluting samples, reference materials, and control materials through the use of programmed probes that perform a wide range of serial and nonserial dilutions; and

5) a temperature controlled housing for storing a plurality of reagent containers, each containing a respective reagent, and a plurality of sample collection tubes, each containing a fluid sample and presenting an optically readable code, such as a bar code or other equivalent coding, identifying the sample and a test to be performed on the sample. There is also a reagent tracking system that monitors the amount of available fluid in each discrete vial or container of reagent, present as a means for sensing liquid levels, one embodiment being a liquid-level sensor on each probe that aspirates and dispenses liquid.

C. The Optical Inspection Segment.

The optical inspection segment of the automated analyzer consists of three components, 1) a means for multiple wavelength analysis; 2) the use of a broad spectrum of wavelengths; and 3) continuous normalization of the fluctuations in light levels associated with sample to sample variability.

The optical inspection portion of the analyzer is provided for by:

1) and 2) a multichannel optical monitoring system as described in commonly owned US patent 5,002,392, issued on March 26, 1991, and incorporated herein by reference and commonly owned US patent application serial number 07/896,579, "Method for Scanning Photodiodes", also incorporated herein by reference; and
3) fluctuations in light levels associated with sample to sample variability, such as differences in color from sample to sample, are normalized through a quality assurance program prior to data analysis of the test results.

D. The Signal Processing Segment.

The signal processing segment of the analyzer consists of three components: the determination of kinetic endpoints; complex processing that determines endpoints other than clot formation, such as immunological complexes or chromogenic endpoints; and an on-line database against which each test result can be compared.

In greater detail, the signal processing segment of the analyzer consists of:

1) a means for determining kinetic endpoints, for example, a computer program, that is able to determine the rate of acceleration that a clot is forming. This analysis is unique in that it is based on the kinetics of clot formation as opposed to the use of a threshold, which is sensitive to biological, mechanical and electrical artifacts, thereby giving more accurate test results.
2) a means for determining the endpoint of a variety of assays other than those based on clot formation. For example, chromogenic assays such as ATIII and Protein C, and immunologic assays based on the interactions of antigens and antibodies, such as the assay for D-dimer, are read based on color changes or the presence or absence of agglutination or some type of label; and
3) a means for creating and storing reference curves that can be used for the evaluation of controls and patient samples.

Each of the above segments of the coagulation analyzer is integrated with on-line quality control and quality assurance programming. These are conducted to minimize bias and imprecision throughout the analyzer.

E. The Quality Assurance Segment.

The satisfactory performance of any and all clinical laboratory assays depends on an effective quality control or quality assurance program, which controls each of the parameters listed below. Control of these parameters minimizes imprecision associated with random error and minimizes bias associated with systemic error. The parameters controlled by the analyzer are:

A) specimen integrity and handling;
B) reagent and expendable availability and quality;
C) suitability and sensitivity of mechanical metering devices;
D) suitability and sensitivity of reaction inspection and measuring devices;
E) suitability and sensitivity of data analysis methods, wherein statistical quality control rule analysis of the control data allows for the monitoring of the system in statistical control, assuring the validity of the results; and
F) minimized bias when compared to reference methods.

In order to assure accurate laboratory assay results, it has always been necessary to devise quality assurance methods to monitor important variables of each of the above critical parameters. This type of monitoring is equally necessary for manual, semi-automated and automated analytical methods. Prior to the present invention, skilled laboratory workers were responsible for monitoring these important variables for manual and semi-automated analytical methods using rudimentary off-line means. Some of these means included a traditional Levy Jennings approach to control ruling, visual inspection of samples for anomalies and no real-time monitoring of some instrument parameters was available. But the present invention requires on-line monitoring using sophisticated computer programming and integrated means.

Although each type of coagulation laboratory assay has specific critical parameters within the general parameters described above, some additional hemostasis and thrombosis assay critical parameters include:

1) Positive patient identification which includes bar code or similar identifier tracking, Delta check with previous specimen from the same patient; physiologic panic value evaluation; operational comments regarding the sample that follow data to the final report; and a statistical evaluation of replicate tests.

2) Preanalytical Variables are variables that can contribute to an anomalous result. Examples are specimen age, plasma with clot contamination, the amount of anticoagulant present in the blood collection tube to plasma collected ratio, nonanalyte interferences, optimized thermal storage of specimens to offset degradation with activation, hemolysis of sample, bilirubin content, and lipemic samples.

3) Sampling from a primary specimen container that allows for repeated testing from the same closed container without plasma carryover effects or aerosolization of the blood samples.

4) Reagent and Expendable Availability - a broad diagnostic assay menu supported with the proper reagents, which are monitored, tracked and flagged for the operator when the levels are low; a liquid level sensor on the probe via, for example, capacitance touch facilities; flagging failure of the analyzer to dispense; logic programming omitting the performance of an assay if sufficient reagents not available; bar code or similar identifier identification of the actual placement of a reagent in reagent tray; and preloaded cuvette cassettes handling a large number of cuvettes insure the optical clarity of the cuvette by minimizing handling of cuvettes.

5) Reagent quality is insured by refrigerated storage; by a reagent tray cover that minimizes evaporation and condensation; by the tracking of expiration dates; by tracking of reagent quality within each run, day-to-day, and month-to-month via an on-board quality control program using controls; by an assay specific quality control program which employs statistical rules with the ability to detect errors in the reagents; by tracking reagent quality independently of biological control plasmas using averaged patient data parameters; by normalizing minor drifts in reagent viability over time by assay calibration using calibrator plasmas; and by stirring reagents requiring stirring to maintain homogeneous suspension.

As can be seen from the above descriptions of the various segments of the automated coagulation analyzer, the segments are interdependent. Each of the four segments, specimen handling, sample preparation, optical inspection and signal processing are monitored and regulated and checked by on-board quality assurance and quality control program, providing an oversight function for all critical parameters. Each of these segments will be now more fully discussed along with the integration of these segments with the quality control and quality assurance features of the automated coagulation analyzer.

## II. Specimen Handling

The present invention automatically handles the sample to be tested from the moment the collection tube containing the sample is placed in the analyzer. The analyzer has a first transporting device for transporting the sample collection tubes, in order, first to the programming station and then to the sample insertion station and a second transporting device for transporting the cuvettes through the sample insertion station, the reagent station and on to the optical monitoring device where the optical characteristics of the reaction volume in the respective reaction wells can be monitored.

According to a preferred embodiment of the invention, the sample collection tubes are evacuated and sealed by a septum, the tubes are sampled with a piercing/aspirating sample probe which deposits the proper amount of sample into the well of a cuvette. The preferred cuvette is described in Karp et al. US Des. 325,090 and US 5,040,894.

According to another aspect of the invention, the temperature controlled housing maintains the temperature of the evacuated collection tubes and the reagent containers between 9°C and 15°C.

Further, the second transporting device preferably includes a linear track for guiding the cuvettes and a drive mechanism for periodically moving the cuvettes along the track in discrete increments. Preferably, the drive mechanism includes a lead screw and the cuvettes are each shaped for engaging the lead screw for being driven along the linear track in the manner described in the above referenced US 5,040,894. According to yet a further aspect of the invention, the cuvette storage includes a device for removing the cuvettes from the storage and placing the cuvettes onto the linear track.

Additionally, the first transporting device preferably includes a plurality of shuttles each for holding a plurality of sample collection tubes and means for moving the shuttles through the programming and sample insertion stations.

Each sample is given a machine-readable identification, such as a bar code, which is used in assay entry and during tracking of the assay. For example, a freshly drawn tube of blood is manually labelled with a bar code, and the bar code, patient identification and required tests are manually entered into the analyzer's computer. The tube is then placed into a shuttle and the shuttle placed in the shuttle storage area, where its bar code is automatically read by the analyzer and tracked by the quality control programming. The septum is pierced with the sampling probe, called Probe 1, which has been programmed as defined in the adf, to withdraw a specified amount of sample from the tube or collection device, which proceeds to aspirate the sample and dispense it into a predetermined well of a predetermined cuvette. The probe is then washed in order to remove essentially all of the sample remaining on it, in order to avoid cross-contaminating the next sample. Wash solutions include, among others, water, bleach solutions, preferably a 10% bleach solution, or specifically formulated wash solutions that are capable of removing essentially all of the sample from the probe. At times later in the assay, each probe can be washed after each use, for the same reasons. However, another reason for washing a probe is because in these types of assays, should thrombin, thromboplastin or phospholipids contaminate the probe, they are extremely difficult to remove, and thereby contaminate the next assay well. Removal of these extremely sticky substances from the probe is needed for both the method and the instrument to perform.

The analyzer has been programmed so that this particular well will have a specific assay performed in it. The quality assurance program tracks the well at specified times throughout the test procedure, confirming assay identification, correct volume delivery, correct adf interpretation and proper temperature control at critical times.

Preanalytical variables, such as the presence of hemolysis, bilirubin, lipemia, and fibrin clots are determined in conjunction with the performance of an assay. This is accomplished by utilizing a bichromatic technique inspecting a baseline wavelength and wavelength where the substance of interest can be detected. Unique algorithms are then applied to quantitate each substance. For example, hemolysis is detected by monitoring transmittance at 535nm and 515nm and computing a hemolysis index, which is

$$\text{Hemolysis Index (HI)} = \frac{\text{Normalized transmittance at 535nm}}{\text{Normalized transmittance at 515nm}}$$

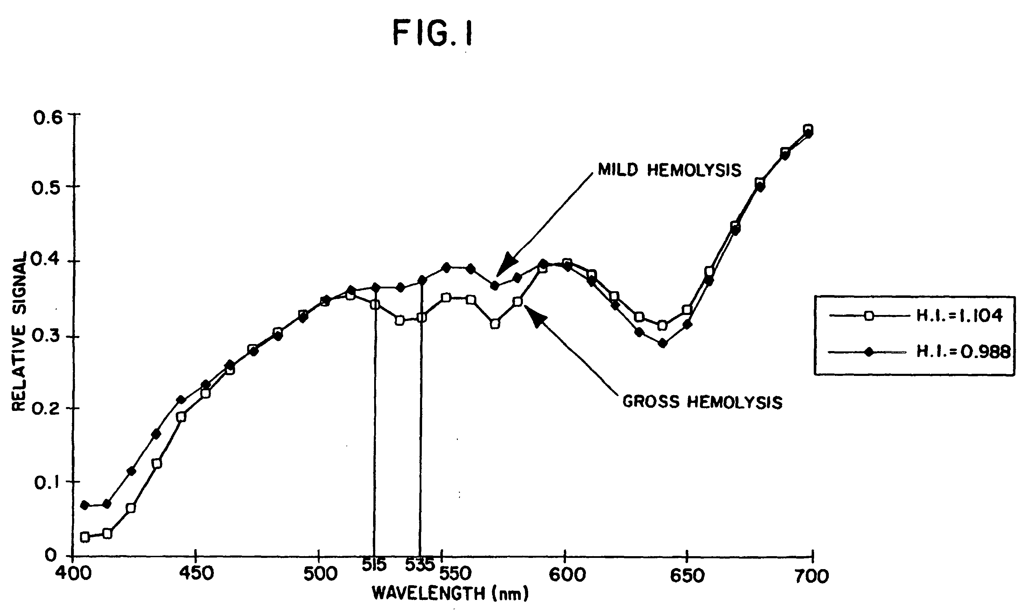

The heme unit of the hemoglobin molecule has adsorption at the 535nm band pass (see Figure 1).

Bilirubin is conducted the same way but uses 450nm and 710nm as the wavelengths of interest, while lipemia is measured by monitoring the normalized transmittance at 710nm.

### III. Sample Preparation

The next step is the automatic preparation of the sample for testing. This includes the ability of the analyzer to access any reagent and to deposit it in a cuvette well; to wash the probe through which the reagent is accessed after each reagent is dispensed with a wash solution as described above in order to avoid cross-contamination problems between reagents or reagents and samples; a universal profile testing method for all coagulation assays; a means for automatically diluting the sample; and a means for monitoring levels of the reagents and samples in their cuvettes and tubes.

The random access movement of the probes is used to aspirate and dispense reagents and samples according to test protocol for a particular assay (defined by the adf parameters), ordered or scheduled from the bar code of a given sample collection tube. This movement, and a description of the analyzers instrumentation, is more fully explained in the co-pending parent application, USSN 07/833,950. Correct reagent/plasma volume delivery is monitored through the use of a novel dye tracking system, one type of which is described in U. S. patent number 5,068,181. The versatility provided by the presence of adf's allow the fully automated analyzer to be optimized for each specific assay, allowing for the flexibility required for radically different assay formats.

Below are diagrams of adfs for three assays (Tables 1-3), Factor VIII reference curves, PT and plasminogen reference curves. The first column notations A1, A2, A3 and A4 refer to the automated arms of the analyzer, the second column notations refer to various operations, such as pumping velocity, dilution, and aspiration. The numbers after the colons are the editable parameters, dealing with each of the various operations, such as volume amount or number of dilutions or velocity. A number of 000 means that no function is performed by the particular arm at that particular point in time. The notations after A4 refer to the optical set-up of the analyzer.

TABLE 1 -

| adf Parameters for FVIII Ref. Curves | | | |
|---|---|---|---|
| A1 Run Volume | 50 | A3 Reagent Volume | 051 |
| A1 Pump Velocity | 1000 | A3 Reagent Airslug : | 040 |
| A1 Sample Location | 001 | A3 Pump Velocity | 1250 |
| A1 Sampling Macro | SFSTASP | A3 Reagent Location : | 001 |
| A1 Dilution Count | 007 | A3 Fetch Macro | FETCH2 |
| A1 Dilution Mix (1:x) | 010 | A3 Deliver Macro | DELREAGENT |
| A1 Dilution Sample | 010 | A3 Mash/Rinse Macro : | WASH |
| A1 Dilution Primer | 090 | A3 Cleaner Volume | 005 |
| A1 Dilution Aspirate | 050 | A3 Cleaner AirSlug : | 085 |
| A1 Dilution Macro | SMIDASP | A3 Rinse Volume | 800 |

TABLE 1 -   (continued)

| adf Parameters for FVIII Ref. Curves | | | |
|---|---|---|---|
| A1 Non-Serial Dilution Count | 000 | | |
| A1 Wash/Rinse Macro | RINSEP | A4 Reagent Volume | 051 |
| A1 Cleaner Volume | 000 | A4 Reagent AirSlug | 040 |
| A1 Cleaner AirSlug | 000 | A4 Pump Velocity | 1500 |
| A1 Rinse Volume | 800 | A4 Reagent Location : | 002 |
| | | A4 Fetch Macro | FETCH2 |
| A2 Dilution Volume | 051 | A4 Deliver Macro | DELOPTICS |
| A2 Primer Volume | 000 | A4 Wash/Rinse Macro | WASH |
| A2 Aspirate Volume | 000 | A4 Cleaner Volume | 005 |
| A2 Dilution AirSlug | 010 | A4 Cleaner AirSlug | 085 |
| A2 Pump Velocity | 1000 | A4 Rinse Volume | 800 |
| A2 Buffer Location | 005 | Assay Blank time | 007 |
| A2 Dilution Macro | FETCHOEL | Assay Maximum Time | 240 |
| A2 Dilution Count | 000 | Normalization Value | 3000 |
| A2 Dilution Concentrat | 000 | Replicate Count | 000 |
| A2 Dilution Mix (1:x) | 000 | Wavelength Count | 003 |
| A2 Non-Serial Dilution Count | 000 | 5, 15, 20 | |
| | | Dltn Recalc Override | 0000 |
| A2 Throw Away Volume | 000 | Spare 2 | 0000 |
| A2 Middle Dilution | 000 | Spare 3 | 0000 |
| A2 Middle Primer Vol | 000 | Spare 4 | 0000 |
| A2 Middle Aspirate Vol | 000 | Spare 5 | 0000 |
| A2 Middle Dilution Macro | | | |
| A2 Wash/Rinse Macro | RINSEP | | |
| A2 Cleaner Volume | 000 | ADF Name | FVIIIR.ADF |
| A2 Cleaner AirSlug | 000 | | |
| A2 Rinse Volume | 800 | | |

TABLE 2 -

| adf Parameters for PT | | | |
|---|---|---|---|
| A1 Run Volume | 51 | A3 Reagent Volume | 000 |
| A1 Pump Velocity | 1000 | A3 Reagent Airslug | 000 |
| A1 Sample Location | 001 | A3 Pump Velocity | 0000 |
| A1 Sampling Macro | PIEREC | A3 Reagent Location | 000 |
| A1 Dilution Count | 000 | A3 Fetch Macro | |
| A1 Dilution Mix (1:x) | 000 | A3 Deliver Macro | |
| A1 Dilution Sample | 000 | A3 Wash/Rinse Macro | |
| A1 Dilution Primer | 000 | A3 Cleaner Volume | 000 |
| A1 Dilution Aspirate | 000 | A3 Cleaner AirSlug | 000 |
| A1 Dilution Macro | | A3 Rinse Volume | 000 |
| A1 Non-Serial Dilution Count | 000 | | |
| A1 Wash/Rinse Macro | RINSEP | A4 Reagent Volume | 101 |
| A1 Cleaner Volume | 000 | A4 Reagent AirSlug | 040 |
| A1 Cleaner AirSlug | 000 | A4 Pump Velocity | 1250 |
| A1 Rinse Volume | 800 | A4 Reagent Location | 001 |

TABLE 2 -   (continued)

| adf Parameters for PT | | | |
|---|---|---|---|
| | | A4 Fetch Macro | FETCH3 |
| A2 Dilution Volume | 000 | A4 Deliver Macro | DELOPTICS3 |
| A2 Primer Volume | 000 | A4 Wash/Rinse Macro | WASH1 |
| A2 Aspirate Volume | 000 | A4 Cleaner Volume | 030 |
| A2 Dilution AirSlug | 000 | A4 Cleaner AirSlug | 115 |
| A2 Pump Velocity | 0000 | A4 Rinse Volume | 800 |
| A2 Buffer Location | 000 | Assay Blank time | 005 |
| A2 Dilution Macro | | Assay Maximum Time | 150 |
| A2 Dilution Count | 000 | Normalization Value | 3000 |
| A2 Dilution Concentrat | 000 | Replicate Count | 000 |
| A2 Dilution Mix (1:x) | 000 | Wavelength Count | 003 |
| A2 Non-Serial Dilution Count | 000 | 5, 15, 20 | |
| | | | |
| | | Dltn Recalc Override | 0000 |
| A2 Throw Away Volume | 000 | Spare 2 | 0000 |
| A2 Middle Dilution | 000 | Spare 3 | 0000 |
| A2 Middle Primer Vol | 000 | Spare 4 | 0000 |
| A2 Middle Aspirate Vol | 000 | Spare 5 | 0000 |
| A2 Middle Dilution Macro | | | |
| A2 Wash/Rinse Macro | | | |
| A2 Cleaner Volume | 000 | ADF Name | PT.ADF |
| A2 Cleaner AirSlug | 000 | | |
| A2 Rinse Volume | 000 | | |

TABLE 3 -

| adf Parameters for Plasminogen | | | |
|---|---|---|---|
| A1 Run Volume | 50 | A3 Reagent Volume | 050 |
| A1 Pump Velocity | 1500 | A3 Reagent Airslug | 005 |
| A1 Sample Location | 001 | A3 Pump velocity | 1250 |
| A1 Sampling Macro | SFSTASP2 | A3 Reagent Location | 004 |
| A1 Dilution Count | 000 | A3 Fetch Macro | FETCH1 |
| A1 Dilution Mix (1:x) | 000 | A3 Deliver Macro | DELREAGENT |
| A1 Dilution Sample | 010 | A3 Wash/Rinse Macro : | WASH |
| A1 Dilution Primer | 010 | A3 Cleaner Volume | 010 |
| A1 Dilution Aspirate | 000 | A3 Cleaner AirSlug | 045 |
| A1 Dilution Macro | | A3 Rinse Volume | 800 |
| A1 Non-Serial Dilution Count | 000 | | |
| | | | |
| A1 Wash/Rinse Macro | RINSEP | A4 Reagent Volume | 050 |
| A1 Cleaner Volume | 000 | A4 Reagent AirSlug | 040 |
| A1 Cleaner AirSlug | 000 | A4 Pump Velocity | 1500 |
| A1 Rinse Volume | 800 | A4 Reagent Location | 004 |
| | | A4 Fetch Macro | FETCH1 |
| A2 Dilution Volume | 040 | A4 Deliver Macro | DELOPTICS |
| A2 Primer Volume | 140 | A4 Wash/Rinse Macro | WASH |
| A2 Aspirate Volume | 155 | A4 Cleaner Volume | 010 |
| A2 Dilution AirSlug | 000 | A4 Cleaner AirSlug | 080 |
| A2 Pump Velocity | 1600 | A4 Rinse Volume | 800 |

TABLE 3 -   (continued)

| adf Parameters for Plasminogen | | | |
|---|---|---|---|
| A2 Buffer Location | 003 | Assay Blank time | 005 |
| A2 Dilution Macro | BFSTRA | Assay Maximum Time | 060 |
| A2 Dilution Count | 002 | Normalization Value | 3000 |
| A2 Dilution Concentrat | 005 | Replicate Count | 000 |
| A2 Dilution Mix (1:x) | 020 | Wavelength Count | 003 |
| A2 Non-Serial Dilution Count | 001 | 2, 3, 34 | |
| | | Dltn Recalc Override | 0001 |
| A2 Throw Away Volume | 100 | Spare 2 | 0000 |
| A2 Middle Dilution | 010 | Spare 3 | 0000 |
| A2 Middle Primer Vol | 040 | Spare 4 | 0000 |
| A2 Middle Aspirate Vol | 050 | Spare 5 | 0000 |
| A2 Middle Dilution Macro | BMIDASP | | |
| A2 Wash/Rinse Macro | RINSEC | | |
| A2 Cleaner Volume | 000 | | |
| A2 Cleaner AirSlug | 000 | | |
| A2 Rinse Volume | 800 | | |

The universal testing profile is an extremely important part of this invention. All known coagulation analyzers, semi-automated and automated, perform on the basis that each test requires a unique profile. For example, only Prothrombin (PT) or only APTT assays are to be run at any given time as a batch, and therefore such machines are programmed to run the same way for each sample. They are incapable of doing a prothrombin time test on one sample and an APTT on the next, as the temperature vs. time parameters of each test are different. Some analyzers can perform multiple batch analysis simultaneously, but do not use the same profile. One way of doing so is being having unique pathways for each batch mode. The universal thrombosis and hemostasis temperature profile (temperature vs. time) is a method that allows all coagulation assays to be run through the same linear transport system using the same timing sequence, on the analyzer, to produce the necessary profile, with variables being the probe delivery temperatures and volumes, which are functions of parameters established by the adf.

The universal profile represents a balance between the different thermal requirements for each assay. This allows coagulation assays to be performed in a continuous, fully automated format.

There are nine critical requirements of the preferred universal thrombosis and hemostasis profile:

1. The temperature of the sample dispensed into the cuvette is preferably between about 4°C and about 25°C.

2. The sample must remain in a temperature range of from about 4°C to about 25°C until the heating sequence begins.

3. The time required to warm the sample temperature from the pre-ramp temperature range to the post-ramp temperature range is 80 ± 10 seconds.

4. The temperature of the sample at the end of the ramp must be 33°C ± 1°C.

5. If the test is an APTT, the activation reagent is added immediately at the end of the ramp (Arm 3).

6. The temperature of the reagent delivered at R1 is 40°C ± 1°C, raising the reaction temperature to 37° C.

7. If the test is a PT, the sample must reach 37°C ± 1°C in 120 ± 10 seconds after the 80 second ramp segment (Arm 4).

8. The temperature of the reagent delivered at R2 is 37°C ± 1°C.

9. The temperature of the mixture must remain at 37°C ± 1°C for at least 360 seconds after R2.

These temperature and time controls occur due to the interaction of the heated probes that aspirate and dispense liquids, which are described in more detail in US 5,178,019 to Keiter, and the portion of the sample handling system, the heating and cooling track, as described in USSN 07/833,950.

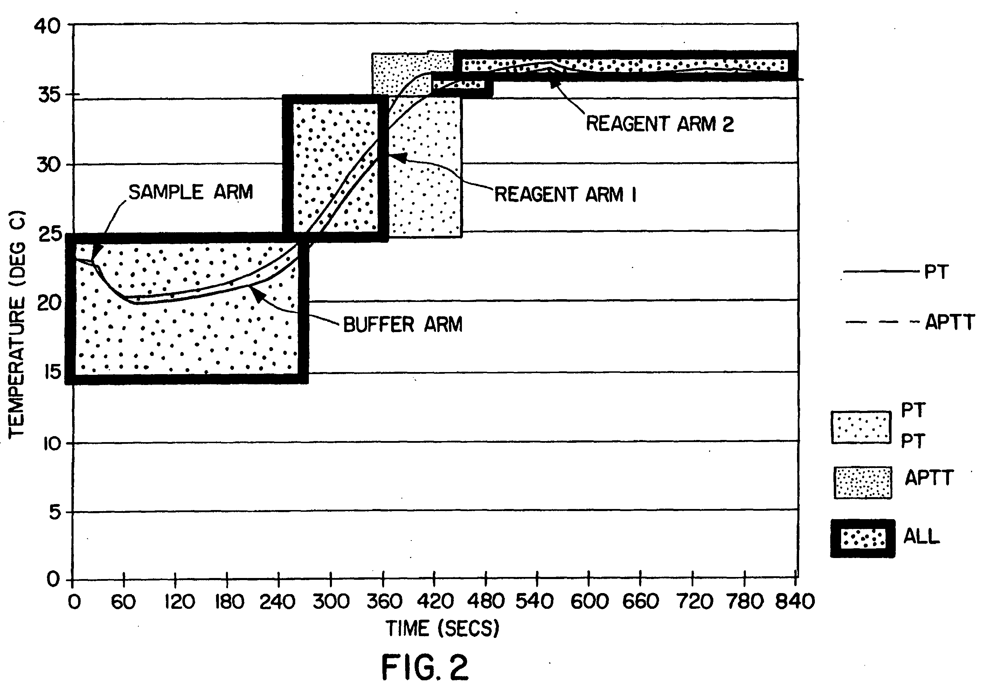

Flexibility in the adf allows for minor changes in temperature profiles that allow for the total optimization of each assay. Diagrams of the profile and optimization ranges for the PT and APTT assays are attached (see Figure 2).

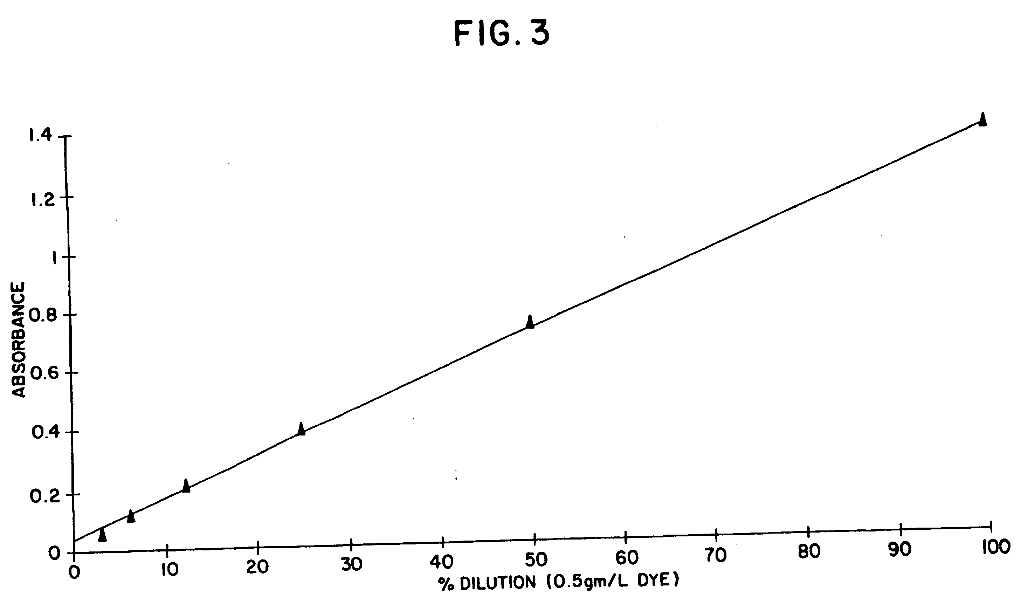

Another aspect of the sample preparation segment of the automatic coagulation analyzer is a means to automatically dilute samples. The analyzer performs a wide range of serial and nonserial dilutions for a large set of assays in a real time format. The adf for each complex method (methods requiring dilutions) defines the dilution: serial and non-serial, which buffers to use, how to dilute concentrated buffers and which arm to perform the dilution on (arm 1 or 2). Successful dilutions are a function of fluidic movement, probe design and robotic design. In addition, the analyzer, with its built-in quality control and quality assurance programming, ensures the accuracy of the dilutions. Figure 3 shows the relative absorbance rates of a dye sample, serially diluted. A least squares regression line is also shown, and its goodness-of-fit measure $r^2$.

Finally, the analyzer provides a means for monitoring levels of the reagents in their containers and of the samples in their sample tubes or holding devices. This is another necessary function for a fully automated analyzer, as an assay cannot be performed without the proper amount of reagents. In the present analyzer, a reagent chamber holds a large number of reagent containers, at a temperature of about $7°C$, and some reagents may be automatically mixed to maintain a homogeneous suspension. If necessary, the reagent is heated in the probe before dispensing. Fluid level sensing is used to control the height of a reagent probe relative to the level of a reagent in its container.

### IV. Optical Inspection

Once the reagents are added to the test sample, the reaction, if any, is read through spectrophotometric means. The optical inspection segment of the analyzer consists of a means for multiple wavelength analysis; a means to continuously normalize the fluctuations in light levels associated with sample to sample variability; and a means for using a broad spectrum of wavelengths in order to read the results of a variety of test reactions at the appropriate wavelength for that test.

As stated above, a preferred format of these functions are well-described in US 5,002,392 and USSN 07/869,579.

The quality control programming insures proper wavelength selection by monitoring the signal noise to ratio for each sample as defined in the adf. If the signal to noise threshold is exceeded then another wavelength is used. If all wavelengths are unacceptable then an error flag is provided to the user.

The quality assurance programming assures that the true wavelength is being evaluated by incorporating a piece of spectral glass with known absorbance characteristics. The peaks are measured by the analyzer and compared to the known values. Furthermore, a liquid crystal clot simulator has been incorporated into the optics module that when properly stimulated creates a signal that is similar to a clot. The output is inspected to insure the integrity of the optics unit as well as the integrity of the analysis algorithms.

### V. Signal Processing

The signal processing segment of the analyzer is the segment that reports the results of the assays. The analyzer has the ability to determine kinetic endpoints; to do multirule analysis of the final test results; to perform complex processing that determines endpoints other than clot formation; and provides an on-line database against which each test result can be compared.

An endpoint algorithm library is required to facilitate the analysis of the different types of assays. The major categories of endpoint analysis are linear rates, logarithmic rates, relative magnitudes and kinetic endpoints based on the biological characteristics of multianalyte mixtures (i.e., clot formation).

For example, the standard way of determining a PT or APTT clotting time is to take a sample, add the appropriate reagents and visually or spectrophotometrically determine when a clot has formed. When visually inspecting a sample for clot formation, the sample will turn more turbid after a clot has formed. Semi-automated analyzers set an endpoint at an arbitrary level of light reduction that is somewhat equitable to the visual method. However, this method is susceptible to error due to the presence of mechanical, optical, electrical and biological artifacts. Furthermore, as the instrument "ages", decreases in light transmission can result in a shift in the endpoint times.

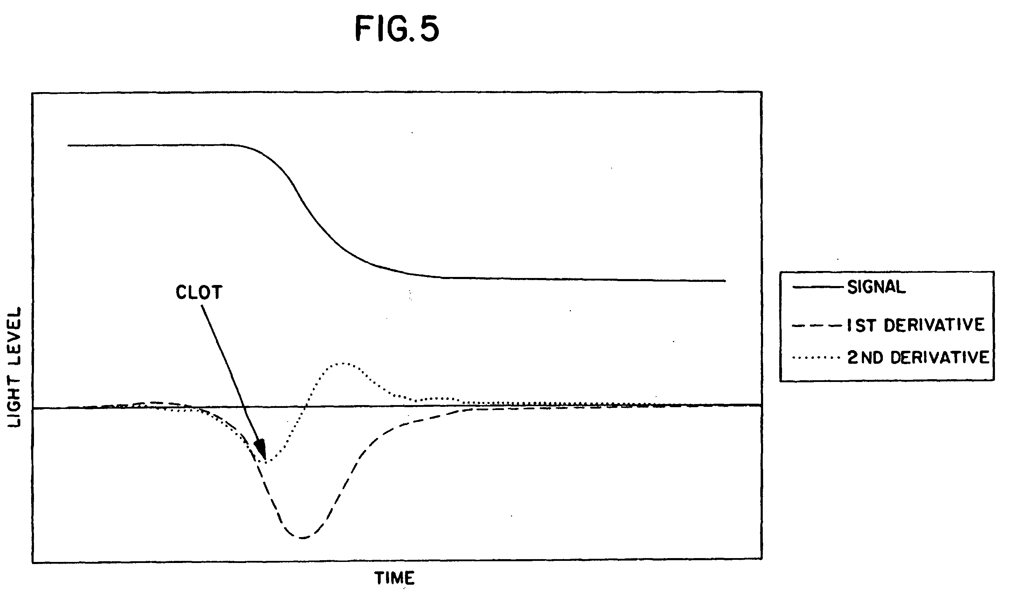

Visual clot formulation occurs when the specimen mixture turns turbid. This can be physiologically associated with the initial conversion of fibrinogen to fibrin. (See, E. Ludvigh, "Perception of Contour," Bureau of Medicine and Surgery, Proj. No. NM001 075.01.04, Aug. 17, 1953.) The maximum rate of acceleration, the time of the peak of the second derivative, is indicative of the initial fibrinogen, fibrin conversion and directly correlates to a visual method (see Figure 5). Because the time is computed on a data stream that is independent of arbitrary thresholds the usual artifacts associated with the instrument and biologies are negated resulting in more accurate and precise results. The same approach of analyzing characteristics of the signal stream for unique parameters is applied to the calculation of rate and magnitude for chromogenic, immunologic and quantitative fibrinogen assays.

For complex assays, kinetic endpoint results, such as clot times and reaction rates, additional processing is required. In these assays, the desired reported value of a sample is a function of some reference curve, which relates a quantity of a known analyte to reported endpoint values. Typically, a series of endpoint-based tests are performed on a series of concentrations from dilutions of a reference material. The paired data sets of recovered and reported values are then used to construct the reference curve.

The reference curve represents a function which is a model built using the sample data. (See, "Computer Evaluation of Reference Curves for the Estimation of Extrinsic Coagulation Factors," Frank et al., <u>Comput Biol Med</u>, Jan 1978). Traditionally, these functions have been assumed to be of a form similar to

$$f(\text{recovered value}) = a_1 g(\text{reported value}) + a_0$$

where f(), g() are typically $\log_{10}()$. This function is particularly easy to represent graphically by hand, and numerically the coefficients can be recovered by simple linear regression using the formulas,

$$\text{slope} = \frac{n\Sigma xy - (\Sigma x)(\Sigma y)}{n\Sigma x^2 - (\Sigma x)^2}$$

$$\text{intercept} = \overline{Y} - \text{slope } \overline{X}$$

This type of function, while seemingly accurate, especially when calculated graphically by hand, is not representative of the true biological processes and leads to errors because points deviating from a straight line on a log-log reference curve are thought to deviate by random error and are neglected.

These simple functions restrict the degrees of freedom necessary to accurate model coagulation assays, which are more complex than simple chemical determinations based on known formulas. There are agents present in addition to the analyte of interest that complicate the cause-effect relationship.

The methods available in this invention allow for more flexibility in designing a model of the reference function which relates the desired reported results with recovered values. These functions provide a more accurate representation of the shape of the reference curve for each assay, accommodating unknown extraneous agents, which leads to more accurate and precise results; also provide robustness in the presence of small errors in the data; gives accurate representation of all sample data, without neglecting points; and providing for an increased range of reference recovery due to incorporation of all dilutions, including those that were once ignored due to deviation from the straight line.

Three methods that are generally used to create models from experimental data are: (1) Gauss Jordan and (2) Singular Value Decomposition (SVD) for linear systems, and (3) Levenberg-Marquardt Method for nonlinear systems. Gauss-Jordan is generally considered a simpler, but less accurate method for solving linear systems. Singular-Value is more costly in terms of number of operations, but it has the advantages of being more accurate, and is robust in the presence of singularities, where Gauss-Jordan cannot function. These singularities are approached when there are two solutions very close together, which is not uncommon. The error creeps into Gauss-Jordan when two very large numbers cancel each other when there is a very small pivot (i.e., close to singular). The accuracy of SVD and Levenberg-Marquardt methods are comparable.

The general form of a linear (in its coefficients) data model is

$$y(x) = \sum_{k=1}^{M} a_k X_k(x).$$

The merit function typically used to determine the best set of parameters $a_k$ is

$$\chi^2 = \sum_{i=1}^{N} \left[ \frac{y_i - \sum_{k=1}^{M} a_k X_k(x_i)}{\sigma_i} \right]^2.$$

The minimum of this function is found where the derivative of $\chi^2$ with respect to all M parameters $a_k=0$, which leads to what are known as the normal equations, or a linear system

$$\sum_{j=1}^{M}\left[\sum_{i=1}^{N}\frac{X_j(x_i)X_k(x_i)}{\sigma_i^2}\right]_{kj} a_j = \left[\sum_{i=1}^{N}\frac{y_i X_k(x_i)}{\sigma_i^2}\right]_k .$$

In order to provide flexibility in determining the optimum reference function for each assay, several components were made available in the complex data analysis module:

- A variety of data transformations, i.e., log10, 1/, etc.;
- switching the x and y variables;
- Polynomials of any order; and
- Piecewise fitting, including overlapping.

The general form of the functions is

$$f(y) = c_0 + c_1 f(x) + c_2 f(x)^2 \cdots c_n f(x)^n$$

where

$$f(x) = x, \frac{1}{x}, \frac{1}{f(x)}, \log(x) \cdots$$

The "piecewise" form is

$$f(y) = \left[f_1(x)\right]_{x=a}^{x=b}, \left[f_2(x)\right]_{x=b}^{x=c}, \cdots \left[f_n(x)\right]_{x=g}^{x=h}$$

where each "subfunction" can be a different type base on any combination of the sample data. Having the ability to select from the above options, an optimum reference function was selected for each complex assay. These optimized reference functions improve both the accuracy and precision of the assays. Each method was tested to determine if samples with known reported values were reported accurately, if diluted samples resulted in reported values at the expected levels, and if the sample data were fit well ($R^2$).

## VI. Quality Assurance

An important part of the quality control package is the use of statistical quality control rules applied to test data. This is very similar to the tests originally utilized for clinical chemistry quality control programs, known as the Westgard multirule systems but that have been applied to coagulation assays (See, Westgard, J., Wiebe, D., "Cholesterol Operational Process Specifications for Assuring the Quality Required by CLIA Proficiency Testing," Clinical Chemistry, Vol. 37, No. 11, pp. 1938-1944, 1991; and Westgard, J., Peterson, P., Wiebe, D., "Laboratory Process Specifications for Assuring Quality in the U.S. National Cholesterol Education Program," Clinical Chemistry, Vol. 37, No. 5, pp 656-661, 1991). This is available for use in two portions of the analyzer, the system quality control program and the on-line test quality control.

The system quality control defaults to a new control run mode when the allotted time designated for control run frequency is reached. The system pauses for the control run, which can only be overridden when an emergency sample is requested. Once the controls are assayed, the statistical quality control rule chosen is activated to interpret the results of the controls. If data passes the rules, quality control results are printed on a hard copy while being simultaneously saved to the on-board computer. If data violates any of the rules, it is flagged through a hard copy and the analyzer's monitor. The user has an option to either repeat the control, troubleshoot the cause of the flag or accept flagged control values.

A preferred variation of statistical rule data structure is shown in the following Table 4.

## Table 4. Statistical Rules Data Structure

The purpose of the statistical quality control rules is to take an array of raw data (which may include data from several different controls or levels) and apply a set of tests to it. The set of tests, if chosen properly can have a very low false rejection rate coupled with a very high probability of rejection of bad results. Before the tests are applied to the data, they are converted to z-scores (normalized) using the level data, allowing different controls to be analyzed together. There can be any number of tests in a statistical rule, and a pair of results is returned for each individual test: one based on clinically significant ranges and one based on statistically significant ranges. Any number of raw data points may be used, as the rules assume that all of the data is to be examined. If GROUP window_flag is selected, the data will be divided into n/N groups. If SLIDING window_flag is chosen, the data will be arranged into (n-N) groups, each group starting at one point past the previous. One test result is returned for all groups - if one group fails, FAILED is the result returned; if all groups pass, PASSED is the result returned. In the case of GROUP and n not divisible by N, the last remaining points are ignored. All data selection is done external to multirules. If there is a "missing point" or if "historical data" is required, it is taken care of before the function is called, in the database query, or its equivalent. In the unexpected case of a set of rules having N's without a common denominator, a group of statistical rules can be used. This can be accomplished by building an object with multiple member statistical rules that returns the result of the cumulative result of its members.

The operation of the sample handling system will now be described in the context of the following specific implementations of the invention, it being understood that the invention is not limited to these particular implementations.

Example I. Mechanical Operation of the Analyzer.

An example of the operation of a preferred analyzer is the following. Operation of the sample handling system according to the invention is centered on a linear track along which cuvettes are advanced from station to station by a lead screw. The basic timing and sequencing of the system is based on advancing the cuvettes along the linear track a distance equal to the distance between successive reaction wells.

Initially, an operator loads cuvettes into the instrument by placing a cassette of cuvettes into a cassette frame. Each cassette holds, for example, 120 cuvettes. The cuvettes are automatically moved from the cassette onto a loading ramp, thus allowing for uninterrupted flow of cuvettes while a cassette is being added to the system. Cuvettes are loaded from the ramp onto the linear track as scheduled, by an arm where they engage the lead screw. Each cuvette preferably has four 1/4 inch reaction wells. The lead screw is activated every twenty seconds to move the cuvettes in 0.25 inch increments in 0.2 seconds. The instrument controller monitors each cuvette by the timing associated with the lead screw. The lead screw advances the cuvettes to the first station, i.e., the sample insertion station, where a sample is delivered to a reaction well aligned with the sample probe. After delivering the sample, the probe is automatically washed. Two minutes and 40 seconds later, the reaction well of the cuvette arrives at the first reagent delivery probe where diluent or a reagent is added, depending on the test being carried out. The probe is automatically washed after each delivery with a washing agent that is correct for removing that particular reagent. The second reagent probe is located two minutes and forty seconds after the first reagent probe, where an activator can be added. Three minutes and forty seconds later the loaded reaction well of the cuvette reaches the third reagent probe where a reagent is added and the reaction monitoring begins. The reaction is monitored electro-optically by an optical monitoring system, which measures changes in the optical transmission of the reaction volume as the clot forms or as the chromometric reaction proceeds. As the cuvette is moved along the track, the optical monitoring continues for twenty consecutive stations, that is, for 300 seconds. Following the optical monitoring station the cuvette leaves the track and is sent to a waste container.

Patient plasma samples are stored in a refrigerated housing in the original evacuated blood collection tubes used to obtain the patient's sample which has been previously spun down to obtain the plasma and bar coded for patient identification and test protocol to be performed. The evacuated sample collection tubes are placed in the holders of shuttles and advanced by the shuttle drive mechanism to the bar code reader. The evacuated sample collection tubes can be arranged in any order since the bar code on each sample collection tube allows the instrument to automatically correlate a patient with a given sample. The bar code read by bar code reader also programs the instrument controller for determining the amount of sample to be aspirated by the sample probe, the number of reaction wells to be filled with the sample, and the amounts and types of reagents/buffers/additives/activators to be injected into the respective reaction wells by multiple reagent probes. Subsequent to the programming station, a sample collection tube is advanced to a piercer where the piercing tube is caused to pierce the septum of the evacuated sample tube to allow the sample probe to be lowered into the sample collection tube to aspirate a programmed amount of sample. The sample probe is next removed from the evacuated sample collection tube and horizontally moved over a reaction well positioned at the sample insertion station and lowered into the reaction well where a programmed amount of sample is expelled into the reaction well. The evacuated sample collection tubes can be removed from the refrigerated housing at any time after sample aspiration is complete; however, because the samples are maintained at lowered temperatures, they can be retained for further testing without having to be immediately removed from its shuttle. The reagent chamber stores various controls, diluents, activators and reagents. In one implementation of the system up to twenty-two containers of these materials are stored in the reagent chamber. All containers are held to a temperature of about 9 - 15° C and the reagents are heated, if necessary, in the reagent probe as they are being dispensed.

pumping in all cases is performed with positive displacement syringe pumps operatively connected with respective ones of the probe. No manipulation of pump tubing is required as is the case with peristaltic pumps. A reagent is dispensed into a reaction well in a manner that promotes mixing with the sample and other contents of the reaction well. The reagent temperature and volume are controlled by the instrument controller.

Desirably, fluid level sensing is utilized to control the height of a reagent probe relative to the level of a reagent in its container and relative to the contents of a reaction well. This permits bringing the outside of a probe into contact with a minimum quantity of reagent. This, in turn, reduces the possibility for carry-over. Additionally, level sensing is used to control the height of a probe above the fluid level while dispensing in order to minimize carry-over and to maximize mixing.

At the same time that all of the above mechanical and programmed functions are occurring, various quality assurance/quality control checks are being automatically performed. Also, data calculations are being automatically performed in order to produce and deliver a final result of the parameter being assayed for. This is shown in the following example.

Example II. Example for Factor VIII on an Automated Analyzer

A Factor VIII assay was conducted to determine the relative quantity of the intrinsic pathway cofactor VIII (FVIII). FVIII concentration is critical in clot formation and deficiencies of it are symptomatic of a hemophiliac condition. It is important to be able to accurately quantitative the FVIII concentration in patient specimens. The invention addresses all aspects of properly performing the complex FVIII Assay.

A FVIII Assay consists of three types of sample evaluations:

    1. Reference materials to normalize the reagent system.

2. Control materials to assure the quality of the reference curve.

3. Patient samples to aid in the diagnosis and treatment of the patient.

Specimen handling and dilutions (Arm 1)

The Factor VIII assay requires the dilution of sample (be it reference, control or patient) serially from a 1:5 to 1:1280. Each type of material requires a different set of dilutions that can be prescribed at time of sampling. The sample was diluted with Imidazole buffer that was contained in the ARM1 reservoir. The Factor VIII reference curve was and is typically prepared as follows:

1. 90uL of Imidazole was loaded into the syringe from the reservoir from arm 1.
2. The arm moved to the appropriate sample reservoir and aspirated 10uL plasma.
3. The total 100uL was delivered to the cuvette.
4. The probe then loaded 50uL of Imidazole from the reservoir for arm 1.
5. 50 uL of the 100uL was aspirated from the cuvette.
6. The track was incremented forward and the 50uL aspirated from the previous well along with the 50uL of Imidazole were delivered into the new well.
7. Steps 4 through 7 were repeated for each additional serial dilution required.

The end result was a FVIII reference curve with seven serial dilutions from a starting dilution of 1:10. The patient and control samples were diluted in the same fashion. Three serial dilutions were performed on a patient sample starting at 1:20, and 2 dilutions on the control material starting at 1:20. The system allows modification of the initial dilution ratio and the number of serial dilutions on a per patient basis.

Quality Checks: The ability to properly perform dilutions was verified using the dye verification process described in the sample preparation detailed description (See Figure 3). If the plasma volume in the primary reservoirs is insufficient, a level sense error is generated. If insufficient volume for the Imidazole reservoir is detected, an error message is given.

Reagent Addition and Incubation

After the appropriate volume of plasma and buffer has been added to the test well, then the appropriate reagent must be added at the appropriate times to prepare the well for optical inspection.

Addition of factor deficient plasma (Arm 2):

The FVIII assay required the addition of 50uL of Factor deficient material 2 minutes and 40 seconds after arm 1. The well was still maintained at ambient temperature.

Addition of Activator (Arm 3):

The FVIII assay is an APTT based assay (test of the intrinsic pathway) and required the addition of an activator material at arm 3 approximately 2 minutes and 40 seconds after the addition of the factor deficient material. During the 2 minutes and 40 seconds the test well is warmed from ambient temperature to about 33°C, then 50uL of activator was added at 39°C raising the temperature in the test well to 37°C. The activator was stored on the reagent tray at 8°C and mixed automatically to maintain a homogenous suspension.

Addition of Calcium Chloride (Arm 4):

The sample was then incubated for 3 minutes 40 seconds at 37°C. The incubation/activation time is critical for clot based test measuring parameters of the intrinsic pathway. At the end of activation, 50uL of 0.25M calcium chloride was added at 37°C, starting the reaction.

Quality Checks: The correct volumes of reagents delivered was assured by the dye tracking system. The quality of the reagents was assured by using the multirule analysis quality control program defined earlier. The thermal profile was monitored by querying temperature sensors as a test well goes over them. If the temperature is determined to be out of range then an error message is reported to the user.

Optical Inspection

The test well was optically inspected for up to 300 seconds. During this inspection process the test well was exposed to fifteen different optical windows for 20 seconds each. A normalization process was required at the first optical windows that normalizes all other windows to the first. Additionally, the normalization process also removes the differences in light levels associated with variability due to the unique turbidity of each plasma. The data was collected for a unique set of assay specific wavelengths and stored in a data file. The data file also contained the data set required to determine quantities of preanalytical variables and dye concentrations for volume verification.

Quality Checks: There are quality checks for wavelength verification, optical integrity (Liquid Crystal Clot Simulator), and an acceptable signal to noise ratio as an indication of proper normalization.

Data Analysis

Endpoint determinations and calibrations:

All endpoints for the test well data files were computed using the peak of the second derivative described above. The endpoints were standardized using a unique calibration scheme. Systematic bias associated with reagent lot to lot variability and instrument to instrument variability was minimized by calibration of the system using labeled plasma calibrators. For more current coagulation systems, systematic error in the measurement of clotting times is due to instrument thermal and fluidic variation as well as in variation in reagent lot to lot sensitivity. Therefore, reference and therapeutic intervals are specific for each instrument and reagent lot and are routinely redetermined. The analyzer's calibration procedure normalized instrument mechanical variables and reagent inter-lot sensitivity, providing uniform APTT clotting times suitable for direct reporting or for use in standardization methods. The analyzer's APTT assays (including FVIII) were calibrated by replicate analysis of VeriCal Calibration Plasmas™ (Organon Teknika) that have assigned clotting times. Observed values were regressed versus assigned values and the resulting regression coefficients were used to normalize the instrument/reagent system.

Reference Curve Generation

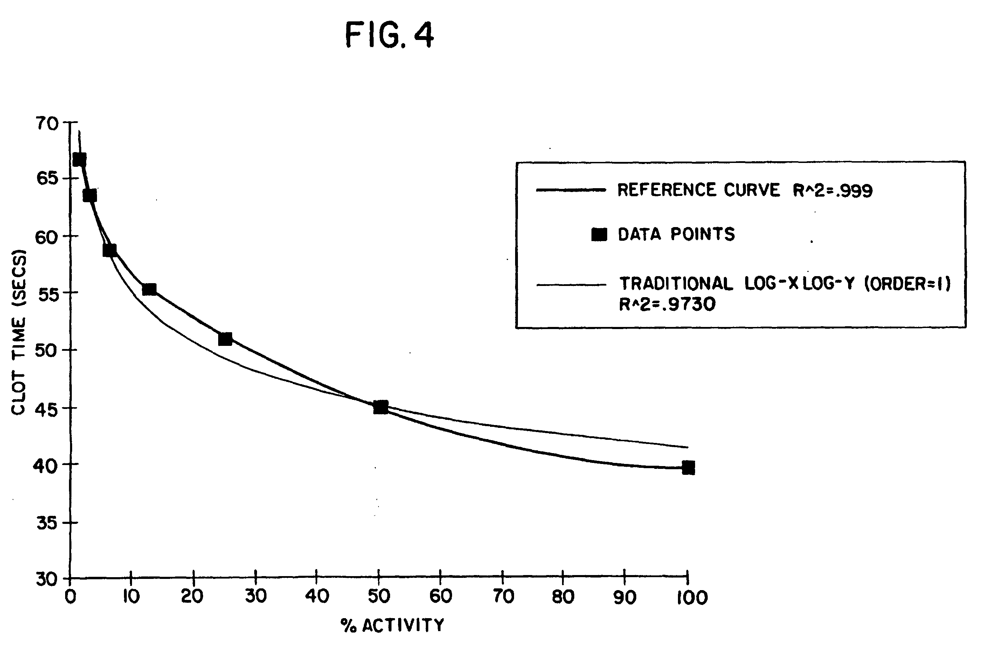

Reference curves were constructed by taking the dilution and known concentration of the reference material and regressing it with the obtained endpoint for each test well. The type of regression and analysis tool used dictates the quality of the reference curve and ultimately the quality of the patient result. The traditional method of constructing a Factor VIII reference curve, log-log transformation, fit to a first order polynomial is not representative of the true biological processes. The curve is better represented by the technique used for this invention. The improvements are most prevalent in the 50 to 100% range and 1 to 10% range, both ranges being clinically significant. The reference curve was constructed by plotting the % activity for the seven dilutions as the independent variable (x), and clotting time as the dependent variable (y). The function is piecewise and discontinuous and composed of two linear sub-curves fit to overlapping sets of data. The first sub-curve uses the first four dilutions and fits a second order polynomial to untransformed data. The second sub-curve fits a second order polynomial to log10 (clot times) and log10 (%activity) to all of the dilutions after the first one. A "cutoff", which determines which sub-curve is used to recover % activity, is established based on the clot time corresponding to the fourth dilution value recovered from the curve. The curve accurately represents the shape of the expected values, and remains robust in the presence of minor variance in the data. Figure 4 shows a comparison between the traditional Factor VIII reference curve and the automatic analysis Factor VIII assay reference curve.

The patient samples were evaluated with respect to a stored or newly generated reference curve and the reported value was corrected for dilution automatically.

Quality Control Checks: Measures of Goodness of fit for the calibration process and the reference curve structure used were defined earlier. The control values were monitored and subjected to the quality control multirule analysis procedures. The slope of the sample dilutions when compared with the controls or reference curve are determined to assess for the presence of Factor VIII inhibitors such as the Bethesda inhibitor.

It will be understood that the above description of the present invention is susceptible to various modifications, changes and adaptations, and the same are intended to be comprehended within the meaning and range of equivalents of the appended claims.

**Claims**

**1.** Method for automatically performing coagulation, chromogenic and immunological assays for hemostasis and thrombosis parameters on a number of samples of plasma, serum or whole blood, the method comprising the steps of:

- identifying a sample and scheduling one or more assays to be performed;
- transferring an aliquot of the sample from a holding device to a test well in a cuvette;
- selecting and adding reagents to the test well;
- moving the aliquot through a heating and cooling track;
- detecting the reaction in the well and computing data from the detection;
- evaluating the computed data to determine the change in or the magnitude of the reaction in the well;
- reporting the results of the evaluation;

   **characterized in that**
   the heating and cooling track has an universal time and temperature profile allowing all assays to be run through the same linear transport system using the same timing sequence; and that each reagent is added to the test well at a specified time and temperature, accommodating the universal time and temperature profile.

2. Method according to claim 2, comprising the steps:

- controlling a cooling unit and maintaining each sample initially within a low temperature range;
- controlling a heating unit that maintains each sample within a predetermined measuring temperature range, which is higher than the initial low temperature range;
- transitioning the temperature of each sample from the initial range to the measuring temperature; and
- reducing the effect of ambient temperature on each sample.

3. Method according to claim 1 or 2, wherein the universal time and temperature profile comprises:

- dispensing the aliquot into the test well at a temperature range from about 4°C to about 25°C and maintaining the aliquot temperature in that range;
- warming the aliquot within $80 \pm 10$ seconds to $33°C \pm 1°C$; and
- immediately adding an activation reagent at a temperature of $40°C \pm 1°C$ and raising the aliquot temperature to 37°C;

   thereby providing a universal time and temperature profile in which an Activated Partial Thromboplastin Time assay can be performed.

4. Method according to claim 1 or 2, wherein the universal time and temperature profile comprises:

- dispensing the aliquot into the test well at a temperature range from about 4°C to about 25°C and maintaining the aliquot temperature in that range;
- warming the aliquot within $80 \pm 10$ seconds to $33°C \pm 1°C$; and
- increasing the aliquot temperature to $37°C \pm 1°C$ within 110 seconds to about 130 seconds, after delivering a reagent into the test well at a temperature of $37°C \pm 1°C$ and maintaining the temperature of the reagent and aliquot at $37°C \pm 1°C$ for a minimum of 360 seconds,

   thereby providing a universal time and temperature profile in which an Prothrombin Time assay can be performed.

5. Method according to any of the preceding claims, wherein evaluating the computed data comprises the steps of:

- calculating a second derivative of a process signal and determining the peak thereof, which is an indication of maximum acceleration of cloth formation; and/or
- calculating a magnitude of change of a process signal; and/or
- calculating a rate of change of a parameter, whereby a first derivative of the parameter is determined.

6. Method according to any of the preceding claims, comprising the step of monitoring and evaluating the sample handling, sample preparation, reaction detection and reported results for quality assurance, wherein the quality assurance comprises checks for sample integrity, reagent integrity, mechanical suitability and optical quality.

**7.** Method according to claim 6, wherein the check for sample integrity comprises detecting at least one preanalytical variable.

**8.** Method according to claim 6 or 7, wherein the check for reagent integrity comprises evaluating control material and applying statistical quality control rules and comparison of a current result to an earlier sample result.

**9.** Method according to any of the claims 6 - 8, wherein the check for system suitability comprises measuring electrical, volumetric and thermal output of critical mechanical components.

**10.** Method according to any of the claims 6 - 9, wherein the check for optical quality comprises monitoring an optical reference clot at an appropriate wavelength and monitoring the signal to noise ratio, thereby insuring the use of acceptable signals.

**11.** Method according to any of the preceding claims, comprising the step of defining a test protocol for each assay.

**12.** Method according to claim 11, wherein the test protocol includes mechanical instructions, optical instructions, data analysis and quality assurance parameters.

**13.** Device for automatically performing the method according to any of the preceding claims comprising:

- programming input means for identifying a sample and scheduling one or more assays to be performed;
- specimen handling means for transferring an aliquot of the sample from a holding device to a test well in a cuvette;
- aliquot preparation means for selecting and adding reagents to the test well;
- means for moving the aliquot through a heating and cooling track;
- detecting means for detecting the reaction in the well and computing data from the detection;
- processing means for evaluating the computed data to determine the change in or the magnitude of the reaction in the well;
- reporting means for reporting the results of the evaluation;

    **characterized in that**
    the heating and cooling track has an universal time and temperature profile allowing all assays to be run through the same linear transport system using the same timing sequence; and that each reagent is added to the test well at a specified time and temperature, accommodating the universal time and temperature profile.

**14.** Device according to claim 13, comprising:

- cooling controlling means for controlling a cooling unit and maintaining each aliquot initially within a low temperature range;
- heater controlling means for controlling a heating unit that maintains each aliquot within a predetermined measuring temperature range, which is higher than the initial low temperature range;
- a transitioning device for transitioning the temperature of each aliquot from the initial range to the measuring temperature; and
- a reducing device for reducing the effect of ambient temperature on each aliquot.

**Patentansprüche**

**1.** Verfahren zum automatischen Ausführen von Gerinnungs-, chromogenen und immunologischen Assays hinsichtlich Hämostase- und Thromboseparametern an einer Anzahl von Proben von Plasma, Serum oder Vollblut, wobei das Verfahren die Schritte umfasst:

- eine Probe zu identifizieren und einen oder mehrere Assays, die ausgeführt werden sollen, zu planen;
- ein Aliquot von der Probe aus einer Haltevorrichtung zu einer Testvertiefung in einer Küvette zu transferieren;
- Reagenzien auszuwählen und zu der Testvertiefung hinzuzugeben;
- das Aliquot entlang einer Erwärmungs- und Abkühlungsbahn zu bewegen;
- die Reaktion in der Vertiefung zu detektieren und Daten aus der Detektion zu berechnen;
- die berechneten Daten auszuwerten, um die Veränderung bei oder die Größe der Reaktion in der Vertiefung zu bestimmen;
- die Ergebnisse der Auswertung zu berichten;

**dadurch gekennzeichnet, dass**
die Erwärmungs- und Abkühlungsbahn ein universelles Zeit- und Temperaturprofil aufweist, welches ermöglicht, dass alle Assays mittels des gleichen linearen Transportsystems unter Verwendung der gleichen zeitlichen Abfolgen durchgeführt werden; und dass jedes Reagens zu der Testvertiefung zu einem spezifizierten Zeitpunkt und bei einer spezifizierten Temperatur, welche an das universelle Zeit- und Temperaturprofil angepasst sind, zugesetzt wird.

2.  Verfahren nach Anspruch 2, welches die Schritte umfasst:

- eine Kühleinheit zu kontrollieren und jede Probe anfänglich innerhalb eines niedrigen Temperaturbereichs zu halten;
- eine Erwärmungseinheit zu kontrollieren, die jede Probe innerhalb eines vorher festgelegten Messtemperaturbereichs, der höher als der anfängliche niedrige Temperaturbereich ist, hält;
- die Temperatur jeder Probe von dem anfänglichen Bereich zu der Messtemperatur übergehen zu lassen; und
- den Effekt der Umgebungstemperatur auf jede Probe zu verringern.

3.  Verfahren nach Anspruch 1 oder 2, wobei das universelle Zeit- und Temperaturprofil umfasst:

- das Aliquot bei einem Temperaturbereich von ungefähr 4°C bis ungefähr 25°C in die Testvertiefung zu verteilen und die Aliquottemperatur in jenem Bereich zu halten;
- das Aliquot innerhalb von 80 ± 10 Sekunden auf 33°C ± 1°C zu erwärmen; und
- unverzüglich ein Aktivierungsreagens bei einer Temperatur von 40°C ± 1°C zuzusetzen und die Aliquottemperatur auf 37°C zu erhöhen;

wodurch ein universelles Zeit- und Temperaturprofil bereitgestellt wird, bei dem ein Assay zur Bestimmung der aktivierten partiellen Thromboplastin-Zeit ausgeführt werden kann.

4.  Verfahren nach Anspruch 1 oder 2, wobei das universelle Zeit- und Temperaturprofil umfasst:

- das Aliquot bei einem Temperaturbereich von ungefähr 4°C bis ungefähr 25°C in die Testvertiefung zu verteilen und die Aliquottemperatur in jenem Bereich zu halten;
- das Aliquot innerhalb von 80 ± 10 Sekunden auf 33°C ± 1°C zu erwärmen; und
- die Aliquottemperatur innerhalb von 110 Sekunden bis ungefähr 130 Sekunden auf 37°C ± 1°C zu erhöhen, nachdem ein Reagens in die Testvertiefung bei einer Temperatur von 37 ± 1°C zugesetzt worden ist, und die Temperatur des Reagens und des Aliquots bei 37°C ± 1°C für ein Minimum von 360 Sekunden zu halten; wodurch ein universelles Zeit- und Temperaturprofil bereitgestellt wird, bei dem ein Assay zur Bestimmung der Prothrombin-Zeit ausgeführt werden kann.

5.  Verfahren nach einem der vorangegangenen Ansprüche, wobei das Auswerten der berechneten Daten die Schritte umfasst:

- Berechnen einer zweiten Ableitung eines Verfahrenssignals und Bestimmen des Peaks davon, der die maximale Beschleunigung der Gerinnselbildung anzeigt; und/oder
- Berechnen einer Größe einer Veränderung eines Verfahrenssignals; und/oder
- Berechnen einer Rate einer Veränderung eines Parameters, wobei eine erste Ableitung des Parameters bestimmt wird.

6.  Verfahren nach einem der vorangegangenen Ansprüche, welches den Schritt umfasst, die Probenhandhabung, Probenvorbereitung, Reaktionsdetektion und die berichteten Ergebnisse hinsichtlich Qualitätssicherung zu überwachen und auszuwerten, wobei die Qualitätssicherung Überprüfungen hinsichtlich Unversehrtheit der Probe, Unversehrtheit der Reagenzien, mechanischer Eignung und optischer Qualität umfasst.

7.  Verfahren nach Anspruch 6, wobei die Überprüfung auf Unversehrtheit der Probe umfasst, wenigstens eine präanalytische Variable zu detektieren.

**8.** Verfahren nach Anspruch 6 oder 7, wobei die Überprüfung auf Unversehrtheit der Reagenzien umfasst, Kontrollmaterial auszuwerten und statistische Qualitätskontrollregeln anzuwenden und einen Vergleich eines gegenwärtig erhaltenen Ergebnisses mit einem früheren Probenergebnis.

**9.** Verfahren nach einem der Ansprüche 6 bis 8, wobei die Überprüfung auf Systemeignung umfasst, elektrische, volumetrische und thermische Ausgabedaten von kritischen mechanischen Komponenten zu messen.

**10.** Verfahren nach einem der Ansprüche 6 bis 9, wobei die Überprüfung auf optische Qualität umfasst, ein optisches Referenzgerinnsel bei einer geeigneten Wellenlänge zu überwachen und das Signal-Rausch-Verhältnis zu überwachen, wodurch die Verwendung von akzeptablen Signalen sichergestellt wird.

**11.** Verfahren nach einem der vorangegangenen Ansprüche, welches den Schritt umfasst, für jeden Assay ein Testprotokoll zu definieren.

**12.** Verfahren nach Anspruch 11, wobei das Testprotokoll mechanische Instruktionen, optische Instruktionen, Datenanalyse- und Qualitätssicherungsparameter umfasst.

**13.** Vorrichtung zum automatischen Ausführen des Verfahrens nach einem der vorangegangenen Ansprüche, umfassend:

- Programmierungseingabemittel zum Identifizieren einer Probe und zur Planung von einem oder mehreren Assays, die ausgeführt werden sollen;
- Probenhandhabungsmittel zum Transferieren eines Aliquots der Probe von einer Haltevorrichtung zu einer Testvertiefung in einer Küvette;
- Aliquotpräparierungsmittel zum Auswählen und Zugeben von Reagenzien zu der Testvertiefung;
- Mittel zum Bewegen des Aliquots entlang einer Erwärmungs- und Abkühlungsbahn;
- Detektionsmittel zum Detektieren der Reaktion in der Vertiefung und zum Berechnen von Daten aus der Detektion;
- Verarbeitungsmittel zum Auswerten der berechneten Daten, um die Veränderung bei oder die Größe der Reaktion in der Vertiefung zu bestimmen;
- Berichtmittel zum Berichten der Ergebnisse der Auswertung;

  **dadurch gekennzeichnet, dass**
  die Erwärmungs- und Abkühlungsbahn ein universelles Zeit- und Temperaturprofil aufweist, welches ermöglicht, dass alle Assays mittels des gleichen linearen Transportsystems unter Verwendung der gleichen zeitlichen Abfolgen durchgeführt werden; und dass jedes Reagens zu der Testvertiefung zu einem spezifizierten Zeitpunkt und bei einer spezifizierten Temperatur, welche an das universelle Zeit- und Temperaturprofil angepasst sind, zugesetzt wird.

**14.** Vorrichtung nach Anspruch 13, umfassend:

- Kühlungskontrollmittel, um eine Kühleinheit zu kontrollieren und jedes Aliquot anfänglich innerhalb eines niedrigen Temperaturbereichs zu halten;
- Erwärmerkontrollmittel, um eine Erwärmungseinheit zu kontrollieren, die jedes Aliquot innerhalb eines vorher festgelegten Messtemperaturbereichs, der höher als der anfängliche niedrige Temperaturbereich ist, hält;
- eine Vorrichtung zum Übergehenlassen, um die Temperatur von jedem Aliquot von dem anfänglichen Bereich zu der Messtemperatur übergehen zu lassen; und
- eine Verringerungsvorrichtung, um den Effekt der Umgebungstemperatur auf jedes Aliquot zu verringern.

## Revendications

**1.** Procédé pour effectuer automatiquement des analyses par coagulation, chromogéniques et immunologiques des paramètres d'hémostase et de thrombose sur un certain nombre d'échantillons de plasma, de sérum ou de sang total, le procédé comprenant les étapes consistant à :

- identifier un échantillon et programmer une ou plusieurs analyses à effectuer;
- transférer une fraction aliquote de l'échantillon d'un dispositif support vers une cupule de test dans une cuvette ;

- sélectionner et ajouter des réactifs au cupule de test ;
- déplacer la fraction aliquote le long d'un chemin de chauffage et de refroidissement ;
- détecter la réaction dans la cupule et rassembler les données à partir de la détection ;
- évaluer les données rassemblées afin de déterminer la variation ou l'intensité de la réaction dans la cupule ;
- rapporter les résultats de l'évaluation ;

**caractérisé en ce que** le chemin de chauffage et de refroidissement a un profil de temps et de températures universel permettant à toutes les analyses d'être effectuées le long du même système de transport linéaire en utilisant la même séquence de temps ; et **en ce que** chaque réactif est ajouté à la cupule de test à un temps et à une température spécifiés, s'adaptant au profil de temps et de températures universel.

2. Procédé selon la revendication 1, comprenant les étapes consistant à :

- commander une unité de refroidissement et maintenir chaque échantillon au départ dans une plage de températures basses ;
- commander une unité de chauffage qui maintient chaque échantillon dans une plage prédéterminée de températures de mesure, qui est plus élevée que la plage initiale de températures basses ;
- faire passer la température de chaque échantillon de la plage initiale à la température de mesure; et
- réduire l'effet de la température ambiante sur chaque échantillon.

3. Procédé selon la revendication 1 ou 2, dans lequel le profil de temps et de températures universel comprend :

- la délivrance de la fraction aliquote dans la cupule de test à une plage de température comprise entre environ 4°C et environ 25°C et le maintien de la température de la fraction aliquote dans cette plage ;
- le chauffage de la fraction aliquote pendant $80 \pm 10$ secondes à $33°C \pm 1°C$ ; et
- l'ajout immédiat d'un réactif d'activation à une température de $40°C \pm 1°C$ et la montée de la température de la fraction à 37°C ;

ce qui produit un profil de temps et de températures universel dans lequel une analyse du temps de thromboplastine partiel activé peut être effectuée.

4. Procédé selon la revendication 1 ou 2, dans lequel le profil de temps et de températures universel comprend :

- la délivrance de la fraction aliquote dans la cupule de test à une plage de température comprise entre environ 4°C et environ 25°C et le maintien de la température de la fraction aliquote dans cette plage ;
- le chauffage de la fraction aliquote pendant $80 \pm 10$ secondes à $33°C \pm 1°C$ ; et
- l'augmentation de la température de la fraction aliquote à $37°C \pm 1°C$ entre 110 secondes et environ 130 secondes, après la délivrance d'un réactif dans la cupule de test à une température de $37°C \pm 1°C$ et le maintien de la température du réactif et de la fraction aliquote à $37°C \pm 1°C$ pendant un minimum de 360 secondes,

ce qui fournit un profil de temps et de températures universel dans lequel une analyse du temps de prothrombine peut être effectuée.

5. Procédé selon l'une quelconque des revendications précédentes, dans lequel l'évaluation des données rassemblées comprend les étapes consistant à :

- calculer une dérivée seconde d'un signal de réaction et déterminer son maximum, qui est une indication d'une accélération maximale de formation de caillot ; et/ou
- calculer une intensité de variation d'un signal de réaction ; et/ou
- calculer un taux de variation d'un paramètre, ce qui fait qu'une dérivée première du paramètre est déterminée.

6. Procédé selon l'une quelconque des revendications précédentes, comprenant l'étape consistant à surveiller et évaluer la manipulation de l'échantillon, la préparation de l'échantillon, la détection de la réaction et les résultats rapportés pour l'assurance qualité, dans lequel l'assurance qualité comprend des vérifications de l'intégrité de l'échantillon, de l'intégrité du réactif, des qualités mécaniques et de la qualité optique.

7. Procédé selon la revendication 6, dans lequel la vérification de l'intégrité de l'échantillon comprend la détection d'au moins une variable préanalytique.

8. Procédé selon la revendication 6 ou 7, dans laquelle la vérification de l'intégrité du réactif comprend l'évaluation du matériau de contrôle et l'application de règles de contrôle de qualité statistiques et la comparaison d'un dernier résultat à un résultat d'échantillon précédent.

9. Procédé selon l'une quelconque des revendications 6 à 8, dans lequel la vérification des qualités du système comprend la mesure de la sortie électrique, volumétrique et thermique des composants mécaniques importants.

10. Procédé selon l'une quelconque des revendications 6 à 9, dans lequel la vérification de la qualité optique comprend la surveillance d'un caillot de référence optique à une longueur d'onde appropriée et la surveillance du rapport signal sur bruit, ce qui garantit l'utilisation de signaux acceptables.

11. Procédé selon l'une quelconque des revendications précédentes, comprenant l'étape de définition d'un protocole de test pour chaque analyse.

12. Procédé selon les revendications 11, dans lequel le protocole de test inclut des instructions mécaniques, des instructions optiques, une analyse de données et des paramètres d'assurance qualité.

13. Dispositif pour effectuer automatiquement le procédé selon l'une quelconque des revendications précédentes comprenant :

    - des moyens d'entrée de programmation pour identifier un échantillon et programmer une ou plusieurs analyses à effectuer ;
    - des moyens de manipulation de spécimen pour transférer une fraction aliquote de l'échantillon d'un dispositif support vers une cupule de test dans une cuvette ;
    - des moyens de préparation de fraction aliquote pour sélectionner et ajouter des réactifs à la cupule de test ;
    - des moyens pour déplacer la fraction le long d'un chemin de chauffage et de refroidissement ;
    - des moyens de détection pour détecter la réaction dans la cupule et rassembler les données à partir de la détection ;
    - des moyens de traitement pour évaluer les données calculées afin de déterminer la variation ou l'intensité de la réaction dans la cupule ;
    - des moyens de rapport pour rapporter les résultats de l'évaluation ;

    **caractérisé en ce que** le chemin de chauffage et de refroidissement a un profil de temps et de températures universel permettant à toutes les analyses d'être effectuées le long du même système de transport linéaire en utilisant la même séquence de temps ; et **en ce que** chaque réactif est ajouté à la cupule de test à un temps et à une température spécifiés, s'adaptant au profile de temps et de températures universel.

14. Dispositif selon la revendication 13, comprenant :

    - des moyens de commande de refroidissement pour commander une unité de refroidissement et maintenir chaque fraction aliquote au départ dans une plage de températures basses ;
    - des moyens de commande de chauffage pour commander une unité de chauffage qui maintient chaque fraction aliquote dans une plage prédéterminée de températures de mesure qui est plus élevée que la plage initiale de températures basses;
    - un dispositif de transition pour faire passer la température de chaque fraction aliquote de la plage initiale à la température de mesure; et
    - un dispositif de réduction pour réduire l'effet de la température ambiante sur chaque fraction aliquote.

# FIG.1

EP 0 714 506 B1

FIG. 2

EP 0 714 506 B1

FIG. 3

## FIG. 4

REFERENCE CURVE R^2=.999

■ DATA POINTS

TRADITIONAL LOG-X LOG-Y (ORDER=1) R^2=.9730

CLOT TIME (SECS)

% ACTIVITY

# FIG.5